Europäisches Patentamt

European Patent Office

Office européen des brevets

(11) Veröffentlichungsnummer: **0 374 909**
**A2**

## EUROPÄISCHE PATENTANMELDUNG

(21) Anmeldenummer: 89123617.6

(22) Anmeldetag: 21.12.89

(51) Int. Cl.5: **C12N 15/12, C12P 21/02, C12N 1/20, C12Q 1/68, A61K 37/02, G01N 33/68, //(C12N1/20,C12R1:19)**

Patentansprüche für folgende Vertragsstaaten:
ES + GR.

(30) Priorität: 23.12.88 DE 3843513

(43) Veröffentlichungstag der Anmeldung:
27.06.90 Patentblatt 90/26

(84) Benannte Vertragsstaaten:
AT BE CH DE ES FR GB GR IT LI LU NL SE

(71) Anmelder: BEHRINGWERKE
Aktiengesellschaft
Postfach 1140
D-3550 Marburg 1(DE)

(72) Erfinder: Grundmann, Ulrich, Dr.
Am Pfahltor 7
D-3551 Lahntal-Grossfelden(DE)
Erfinder: Amann, Egon, Dr.
Sachsenring 8
D-3550 Marburg(DE)

(74) Vertreter: Klein, Otto, Dr. et al
Hoechst AG Zentrale Patentabteilung
Postfach 80 03 20
D-6230 Frankfurt am Main 80(DE)

(54) Für das Plazentaprotein 11 (PP11) kodierende cDNA, ihre Isolierung und Verwendung.

(57) Die Erfindung betrifft die Isolierung des Gens, das für das plazentaspezifische Protein 11 (PP11) kodiert und seine Verwendung zur gentechnischen Herstellung von PP11.

EP 0 374 909 A2

## Für das Plazentaprotein 11 (PP11) kodierende cDNA, ihre Isolierung und Verwendung

Die Erfindung betrifft die Isolierung des Gens, das für das plazentaspezifische Protein 11 (PP11) kodiert und seine Verwendung zur gentechnischen Herstellung von PP11.

PP11 hat nach der Beschreibung in EP-B1-0 029 191 folgende Eigenschaften:

a) einen Kohlenhydratanteil von 3,9 ± 0,9%, bestehend aus 2,6 ± 0,5% Hexosen, 1,0 ± 0,3% Hexosaminen, 0,05 ± 0,03% Fucose und 0,26 ± 0,07% Neuraminsäure;

b) einen Sedimentationskoeffizienten S von 3,5 ± 0,2 S;

c) ein in der Ultrazentrifuge bestimmtes Molekulargewicht von 44 300 ± 6 000;

d) ein im Natriumdodecylsulfat (SDS)-haltigen Polyacrylamidgel bestimmtes Molekulargewicht von 62 000 ± 3 000;

e) einen Extinktionskoeffizienten E (280nm) von 13,4 ± 1,0 und

f) eine elektrophoretische Beweglichkeit im Bereich der $alpha_1$-Globuline sowie die in der Tabelle 1 dargestellte Aminosäurezusammensetzung.

Tab. 1

Aminosäurezusammensetzung von PP11 (Reste pro 100 Reste in Mol-%)

|  |  | VK% (Variations- koeffizient) |
|---|---|---|
| Lysin | 6,26 | 7,00 |
| Histidin | 3,34 | 1,72 |
| Arginin | 3,31 | 5,60 |
| Asparaginsäure | 10,75 | 2,43 |
| Threonin | 3,31 | 10,49 |
| Serin | 9,63 | 1,84 |
| Glutaminsäure | 13,81 | 2,09 |
| Prolin | 4,10 | 4,68 |
| Glycin | 6,23 | 6,26 |
| Alanin | 6,30 | 1,82 |
| Cystin 1/2 | 3,37 | 4,53 |
| Valin | 4,53 | 5,40 |
| Methionin | 1,00 | 26,22 |
| Isoleucin | 3,60 | 2,24 |

| Leucin | 6,74 | 1,05 |
|---|---|---|
| Tyrosin | 5,90 | 3,02 |
| Phenylalanin | 6,06 | 2,52 |
| Tryptophan | 1,66 | 20,87 |

Da die in o.g. Patentschrift beschriebene konventionelle Isolierung von PP11 äußerst mühsam ist, stellte sich deshalb die Aufgabe, das für PP11 kodierende Gen zu isolieren, um die gentechnische Herstellung von PP11 möglich zu machen.

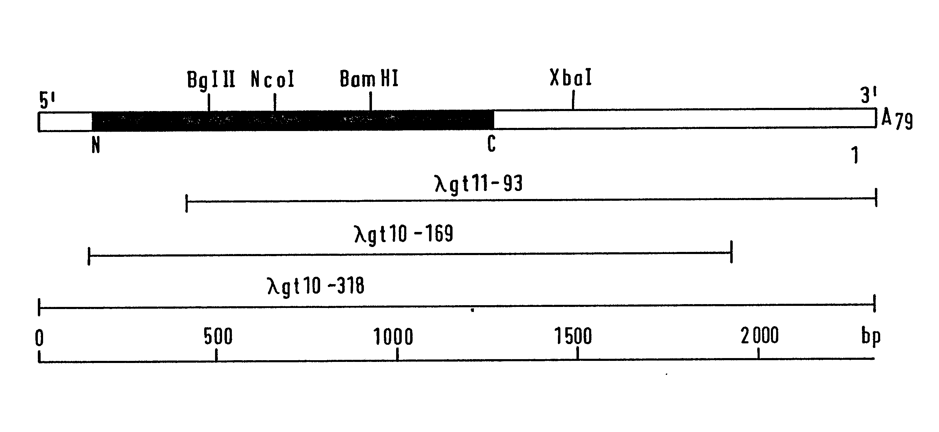

Zunächst wurden mit wie oben beschriebenem konventionell gereinigtem PP11 polyklonale Antikörper erzeugt, die dazu benutzt wurden, um in einer kommerziell erhältlichen cDNA Expressions-Bank von humaner Plazenta (Genofit GmbH, Heidelberg; HL 1008 Human Placenta gt11 Placental tissue, 34 weeks old) nach positiven Klonen zu suchen. Aus 1.2 Millionen rekombinanten Klonen reagiert ein Klon mit dem Antiserum. Er wurde als PP11-93 bezeichnet und enthielt eine Insertion von etwa 1 800 Basenpaaren (bp). Diese Insertion hatte einen offenene Leserahmen von etwa 860 bp, besaß aber kein Start-Methionin. Daraufhin wurde die Insertion von PP11-93 markiert und als Sonde in einer selbst hergestellten Plazenta cDNA Bank in lambda gt10 benutzt. Zwei weitere Klone wurden gefunden (PP11-166 und PP11-169), die mit PP11-93 überlappen und deren Sequenz bestimmt wurde. Klon PP11-169 ist zwar kürzer als Klon PP11-93, aber er überlappt diesen am 5'-Ende um 270 bp und besitzt ein Methionin, das das Start-Methionin sein muß. Mittels eines 5' terminalen 46mer Oligonukleotid des Klons PP11-169 (5' CTCCAACTGGGCACCATGAGGGCCTGCATCTCCCTGGTATTGGCCG) wurde schließlich in dem Klon PP11-318 die gesamte 5'-untranslatierte Sequenz gefunden, die zeigt, daß vor dem o.g. präsumptiven Start-Methionin nur noch Stop-Codons stehen bzw. kein weiteres Methionin auftaucht. Die vollständige cDNA von PP11 ist also einschließlich der Poly(A)-Sequenz 2399 bp lang und besitzt einen offenen Leserahmen für 369 Aminosäuren, was einem Protein von 42 120 D entspricht. Am 5'-Ende sind 154 bp untranslatiert, am 3'-Ende sind 1138 bp untranslatiert und eine 1107 bp kodierende Sequenz wird umschlossen. Die vollständige cDNA-Sequenz ist in der Tabelle 2 aufgelistet, die Position der einzelnen Klone innerhalb der cDNA sowie den kodierenden Bereich zeigt die Figur. "N" steht dort für den N-Terminus, "C" für den C-Terminus des kodierenden Bereichs , "A" für die Poly(A)-Sequenz.

Erfindungsgemäß kann die kodierende cDNA mittels geeigneter Expressionssysteme dazu genutzt werden, PP11 zu exprimieren. Durch die Auswahl des Wirtes kann weiterhin die Form der Modifikation von PP11 beeinflußt werden. So findet in Bakterien keine, in Hefezellen eine andere Glykosylierung als in höheren eukaryotischen Zellen statt. Besonders vorteilhaft erfolgt die Expression von PP11 in E.coli mit dem Expressionsvektor pTrc 99A oder pTrc 99C (siehe Beispiel 11). Es wurde ferner gefunden, daß PP11, vorzugsweise wie oben gentechnisch in E.coli oder Hefe hergestellt, zur Behandlung von Gerinnungsstörungen, Störungen des Complementsystems oder im Zusammenhang mit Störungen der Fibrinolyse eingesetzt werden kann, da es eine amidolytische Aktivität besitzt.

In Kenntnis der Aminosäuresequenz von PP11 ist es möglich, nach konventionellen oder gentechnischen Methoden Aminosäure-Teilsequenzen herzustellen, die als Antigene zur Herstellung von polyklonalen oder monoklonalen Antikörpern dienen. Solche Antikörper sind nicht nur zu diagnostischen Zwecken, sondern auch zur Herstellung von Antikörpersäulen einsetzbar. PP11 kann somit aus Lösungen abgetrennt werden, die es neben anderen Proteinen enthalten. Mit Hilfe der cDNA bzw. Teilen davon kann man auch auf einfache Weise aus einer genomischen Bank den für PP11 kodierenden genomischen Klon isolieren, mit dessen Hilfe nicht nur eine Expression in eukaryotischen Zellen erleichtert wird, sondern auch weitere diagnostische Aussagen möglich sind.

Die Erfindung ist ferner in den Patentansprüchen definiert und in folgenden Beispielen weiter ausgeführt.

Soweit nicht im Text erläutert, werden die folgenden Abkürzungen verwendet:
EDTA = Natrium-ethylendiamin-tetraacetat

SDS = Natrium-dodecylsulfat
DTT = Dithiothreitol
BSA = Rinderserumalbumin

Beispiele:

## 1. Isolierung von RNA aus humaner Plazenta

RNA wurde aus reifer, humaner Plazenta (nach Chirgwin et al., Biochemistry 18 (1979) 5294-5299) gewonnen. Etwa 10 g Plazentagewebe wurden in flüssigem Stickstoff zermörsert, in 80 ml 4 M Guanidinium-Thiocyanat mit 0,1 M Mercaptoethanol suspendiert und 90 sec. bei 20 000 rpm in einem Homogenisator (Ultraturrax) behandelt. Das Lysat wurde 15 min. bei 7 000 rpm zentrifugiert (Sorvall-GSA Rotor) und der Überstand mit 2 ml 1 M Essigsäure und 60ml Ethanol abs. bei -20° C über Nacht gefällt. Nach Sedimentation bei 6 000 rpm und -10° C für 10 min. wurden die Nukleinsäuren in 40 ml 7,5 M Guanidinium-Hydrochlorid (pH 7,0) vollständig gelöst und mit einer Mischung aus 1 ml 1 M Essigsäure und 20 ml Ethanol abs. gefällt. Zur Abtrennung der DNA wurde die Fällung ein weiteres Mal mit jeweils halben Volumina wiederholt. Die RNA wurde in 12ml $H_2O$ gelöst, mit einer Mischung aus 1,2 ml 4 M Kaliumacetat und 24 ml Ethanol abs. gefällt, sedimentiert und schließlich erneut in 10 ml $H_2O$ (1 ml pro g Gewebe) aufgenommen.

## 2. Gewinnung von Poly(A)-haltiger Plazenta-mRNA

Die Plazenta-RNA wurde zur Gewinnung von Poly(A)-haltiger mRNA über Oligo(dT)-Cellulose-Chromatographie (Aviv und Leder, Proc. Natl. Acad. Sci. USA 69 (1973) 1408-1412) in 2 ml Pasteurpipetten in LiCl aufgetrennt. Etwa 5 mg Plazenta-RNA wurden in Puffer 1 (500 mM LiCl, 20 mM Tris (pH 7,5), 1 mM EDTA, 0,1% SDS) auf die Säule aufgetragen. Während die Poly(A)$^+$-RNA an Oligo(dT)-Cellulose gebunden wurde, konnte die Poly(A)$^-$-RNA wieder eluiert werden. Nach einem Waschschritt mit Puffer 2 (100 mM LiCl, 29 mM Tris (pH 7,5), 1 mM EDTA, 0,1% SDS) wurde die Poly(A)$^+$-RNA (Plazenta-mRNA) mit Puffer 3 (5 mM Tris (pH 7,5), 1 mM EDTA, 0,05% SDS) von der Säule eluiert.

Zur weiteren Reinigung wurde die Poly(A)$^+$-RNA auf Puffer 1 eingestellt und erneut über Oligo(dT)-Cellulose chromatographiert. Die Ausbeute an Plazenta Poly(A)$^+$-RNA betrug nach diesem zweiten Reinigungsschritt etwa 4% der eingesetzten RNA.

## 3. Synthese von cDNA aus humaner Plazenta (Plazenta-cDNA) und doppelsträngiger cDNA (dsDNA)

Poly(A)-haltige Plazenta-mRNA wurde vor der cDNA Synthese im 1,5% Agarosegel auf Intaktheit geprüft.

Danach wurden 4 μg Plazenta-mRNA in 65,5 μl $H_2O$ gelöst, 10 min. bei 70° C denaturiert und auf Eis gekühlt.

Die cDNA-Synthese erfolgte in einem 100 μl-Ansatz nach Zugabe von 20 μl RT$_1$-Puffer (250 mM Tris (pH 8,2) bei 42° C, 250 mM KCl, 30 mM $MgCl_2$), 2,5 μl 20 mM dNTP (d.h. aller vier Desoxynukleosidtriphospate), 1 μl Oligo(dT) von 1 μg/ml, 1 μl 1 M DTT, 2 μl RNAsin und 8 μl Reverse Transcriptase (24 U/μl) für 90 min. bei 42° C. Doppelsträngige cDNA (dsDNA) wurde nach Gubler and Hoffmann (Gene 25 (1983) 263-269) synthetisiert. Die Synthese erfolgte unmittelbar nach der cDNA-Synthese durch Zugabe von 305,5 μl $H_2O$, 80 μl RT$_2$-Puffer (100 mM Tris (pH 7,5), 25 mM $MgCl_2$, 500 mM KCl, 50 mM DTT, 250 μg/ml BSA), 2 μl RNase H (2 U/μl), 2,5 μl E.coli DNA Ligase (5 U/μl), 5 μl 15 mM β-NAD, und 5 μl DNA Polymerase I (5 U/μl) und Inkubation für 5 h bei 15° C. Durch Hitzeinaktivierung (70° C, 30 min.) wurde die Reaktion beendet.

Der Reaktionsansatz wurde nach Zugabe von 55 μl 250 μM dNTP, 55 μl 10 mM Tris (pH 7,5), 10 mM $MgCl_2$, 10 μg/ml BSA, 3 μl T4 DNA-Polymerase I (1 U/μl), 2 μl RNase H (2 U/μl) und 2 μl RNase A (2 μg/ml) für weitere 30 min. bei 37° C inkubiert, um die Vollständigkeit der Synthese am zweiten DNA-Strang zu gewährleisten ("Repair Reaction").

4. Ligierung von EcoRI-Linkern an die dsDNA und öffnen der Linker

Zur Errichtung einer Plazenta-cDNA-Bank wurde die dsDNA mit EcoRI-Enden versehen, um sie in die EcoRI-Schnittstelle des Phagenvektors lambda gt10 ligieren zu können (T. Maniatis et al. (1982), Molecular Cloning, A Laboratory Manual, Cold Spring Harbor). Hierzu wurde die dsDNA

    a) mit EcoRI-Methylase behandelt, um interne EcoRI-Schnittstellen der dsDNA zu schützen,
    b) mit EcoRI-Linkern versehen, die
    c) danach mit EcoRI geöffnet wurden.

Zu a):

Die Methylase-Reaktion der dsDNA erfolgte direkt im Anschluß an die "Repair Reaction" nach Zugabe von 25 $\mu$l 500 mM EDTA (pH 8,0), 60 $\mu$l Methylase-Puffer (100 mM NaOAc (pH 5,2), 2 mg S-Adenosyl-L-Methionin) und 2 $\mu$l EcoRI-Methylase (20 U/$\mu$l) durch Inkubation bei 37° C für 30 min. Der Reaktionsansatz wurde mit Phenol extrahiert und die dsDNA mit 60 $\mu$l 4M NaOAc und 1300 $\mu$l Ethanol gefällt. Die dsDNA wurde zweimal mit 70% Ethanol gewaschen, einmal mit Ether ausgeschüttelt und getrocknet.

Zu b):

Die EcoRI-methylierte dsDNA wurde in 88 $\mu$l $H_2O$ gelöst und nach Zugabe von 10 $\mu$l Ligase-Puffer (500 mM Tris (pH 7,4), 100 mM $MgCl_2$, 100 mM DTT, 10 mM Spermidin, 10mM ATP, 1 mg/ml BSA), 1 $\mu$l T4 DNA-Ligase (10 U/$\mu$l) mit 1 $\mu$l EcoRI Linkern (0,5 $\mu$g/$\mu$l) (pGGAATTCC bzw. pAGAATTCT) über Nacht bei 15° C ligiert.

Zu c):

Das Volumen des Ligase-Ansatzes wurde mit 6 $\mu$l $H_2O$, 12 $\mu$l 10x EcoRI-Puffer und 2 $\mu$l EcoRI (120 U/$\mu$l) auf 120 $\mu$l gebracht. Die EcoRI-Verdauung erfolgte für 2 h bei 37° C.

5. Abtrennen nichtgebundener Linker über Kaliumacetat-Gradienten und Größenselektionierung der dsDNA

Zur Abtrennung aller nichtgebundener EcoRI-Linker von der dsDNA wurde der EcoRI-Reaktionsansatz in toto auf einen Kaliumacetat-Gradienten (5-20% KAc, 1 mM EDTA, 1 $\mu$l/ml Ethidiumbromid) aufgetragen und 3 h bei 50 000 rpm und 20° C zentrifugiert (Beckman SW 65-Rotor).

Der Gradient wurde von unten so fraktioniert, daß die ersten fünf Fraktionen 500 $\mu$l maßen und alle restlichen 100 $\mu$l. Die Fraktionen wurden mit 0,01 Volumen Acrylamid (2 mg/ml) und 2,5 Volumen Ethanol gefällt, einmal mit 70%igem Ethanol gewaschen, getrocknet und jeweils in 5 $\mu$l $H_2O$ aufgenommen.

Zur Größenbestimmung der dsDNA wurden 1 $\mu$l jeder Fraktion im 1,5% Agarose-Gel analysiert. Zusätzlich wurde mit 1 $\mu$l jeder Fraktion die Quantität an dsDNA bestimmt.

Fraktionen mit dsDNA über 1 000 bp wurden vereinigt und die Probe so eingeengt, daß die Endkonzentration 27 $\mu$g/ml betrug.

6. Einbau der dsDNA in den Phagenvektor lambda gt10 und "in vitro packaging"-Reaktion

Der Einbau der dsDNA in die EcoRI-Schnittstelle des Phagenvektors lambda gt10 (Vector Cloning Systems, San Diego, CA) erfolgte in einem Ligaseansatz von 4 $\mu$l: 2 $\mu$l dsDNA, 1 $\mu$l lambda gt10 x EcoRI (1 $\mu$g/ml), 0,4 $\mu$l Ligase-Puffer, 0,5 $\mu$l $H_2O$, 0,1 $\mu$l T4 DNA-Ligase. Der Ansatz wurde 4 h bei 15° C inkubiert.

Zur Etablierung der Plazenta-cDNA-Bank im Phagenvektor lambda gt10 folgte mit den lambda-lysogenen Zellextrakten E.coli NS 428 und NS 433 eine "in vitro packaging"-Reaktion des Ligaseansatzes bei Raumtemperatur für 2 h (Vector Cloning Systems, San Diego, CA; Enquist and Sternberg, Methods in Enzymology 68, (1979), 281-298). Die Reaktion wurde mit 500 $\mu$l Suspensionsmedium (SM: 0,1 M NaCl, 8 mM $MgSO_4$, 50 mM Tris (pH 7,5), 0,01% Gelatine) und 2 Tropfen Chloroform gestoppt.

7. Titerbestimmung und Analyse der Plazenta-cDNA-Bank

Die Zahl der "Plaque Forming Units" (PFU) der Plazenta-cDNA-Bank wurde mit kompetenten Zellen des E.coli-K 12-Stammes C600 HFL bestimmt: Sie betrug 1 x $10^6$ PFU. Etwa 80% aller Phagen enthielten DNA-Inserts, die größer als 1 000 Basenpaare waren.

8. Screening einer Expressions-cDNA Bank aus humaner Plazenta mit anti-PP11 Antikörpern

Eine Expressions-cDNA Bank im Phagen lambda gt11 der Fa. Genofit (a.a.O.) wurde mit einer Dichte von etwa 30 000 PFU pro Agarplatte (13,5 cm Durchmesser) ausplattiert. Dazu wurden kompetente Zellen des E.coli Stammes y1090 (ATCC 37197) (R.A. Young and R.W. Davis, Science Vol. 222, 778-782/1983) mit den Phagen 30 min. bei 37°C infiziert und dann auf L-Broth-Platten in Top-Agar ausplattiert. Die Platten wurden 4 h bei 42°C inkubiert, danach mit je einem trockenen Nitrocellulosefilter (Schleicher und Schuell, BA 85, Ref.Nr. 401124) bedeckt. Die Filter waren zuvor mit 10 mM IPTG in Wasser gesättigt worden. Die Platte mit den Filtern wurden erneut 4 h bei 37°C inkubiert. Bevor die Filter wieder abgenommen wurden, wurden Filter und Platte zugleich mit einer rußgeschwärzten Nadel markiert. Die Filter wurden dann in TBST (10 mM Tris-HCl, pH 8,0, 150 mM NaCl, 0,05% Tween 20 und 5% Magermilchpulver) über Nacht bei 4°C inkubiert. Die Filter wurden anschließend noch dreimal für 10 min in TBST bei Raumtemperatur gewaschen und dann mit anti-PP11 Kaninchen Antikörpern in 15 ml TBST pro Filter bei Raumtemperatur 1 h inkubiert. (Die Lösung der Antikörper war zuvor 1:200 verdünnt worden und mit nichtrekombinanten lambda gt11 lysierten E.coli Zellen auf Nitrocellulose Filtern 1 h abgesättigt worden). Nach der Inkubation mit dem primären Antikörper wurden die Filter 4 x 10 min. mit TBST gewaschen. Nunmehr wurden die Filter mit dem sekundären Anti-Kaninchen Antikörper, der mit alkalischer Phosphatase konjugiert war (Fa. Promega, USA - Vertrieb durch Fa. Atlanta, Heidelberg) und zuvor 1:5000 in TBST verdünnt wurde, unter Schütteln 1 h inkubiert. Die Filter wurden danach erneut 4 x 10 min. mit TBST gewaschen. Schließlich erfolgte die Farbreaktion, um die PP11-positiven Klone, an die primärer wie sekundärer Antikörper gebunden waren, unter Reaktion der alkalischen Phosphatase und eines Farbreagenzes (ProtoBlot System der Fa. Protogen) sichtbar zu machen. Pro Farbreaktion wurden für einen Nitrocellulosefilter 15 ml AP Puffer (100 mM Tris-HCl, pH 9,5 100 mM NaCl, 5 mM $MgCl_2$) mit 99 µl NBT-(nitro blau tetrazolium) Substrat (50 mg/ml in 70% Dimethylformamid) und 49,5 µl BCIP(5-Brom-4-Chlor-3-Indolyl-Phosphat)- Substrat (50 mg/ml in 70% Dimethylformamid) versetzt. Die Filter wurden im Dunkeln in der Farblösung etwa 20 min. bis 1 h so lange geschwenkt, bis positive Plaques eine ausreichende Blaufärbung zeigten. Die Farbreaktion wurde beendet durch Eintauchen der Filter in eine Stop-Lösung (20 mM Tris-HCl, pH 8,0 und 5 mM EDTA).

Positive Signale wurden den Plaques auf der entsprechenden Agarplatte zugeordnet. Die Plaques wurden mit einer Pasteurpipette ausgestanzt, in 1 ml SM Puffer (10 mM Tris-HCl pH 7,5, 10 mM $MgCl_2$) resuspendiert und vereinzelt, bis ein einzelner, positiver Plaque vorhanden war. Aus der hier verwendeten Expressionsgenbank wurde ein Klon (PP11-93) gefunden, der jedoch nicht das vollständige Gen enthielt, sondern mit etwa 1800 bp Gesamtlänge der Insertion einen offenen Leserahmen - ohne Start-Methionin - von 860 bp aufwies.

9 a) Herstellung einer PP11-spezifischen Sonde

Nach bekannten Methoden wurde aus dem PP11-93 Klon die cDNA isoliert und wie folgt mit $^{32}$P markiert.

10 ng DNA wurden nach dem "Multiprime DNA labelling System" (Fa. Amersham, Braunschweig) mit Klenow-Polymerase unter Anwesenheit von alpha-$^{32}$P dCTP markiert (eingesetzt wurden 35 µCi/25 µl Reaktionsansatz). Die DNA Sonde hatte eine spezifische Aktivität von 330 MBq/pMol.

9 b) Screening der selbst nach 1. bis 7. hergestellten Plazenta-cDNA Bank mit PP11 spezifischen Sonden

Dazu wurden 3 x $10^4$ PFU mit Zellen des E.coli K 12-Stammes C 600 HFL in Weich-Agar auf 13,5 cm Petrischalen ausplattiert und 6 h bei 37°C inkubiert. Zu diesem Zeitpunkt war noch keine vollständige Lyse eingetreten. Die Platten wurden über Nacht im Kühlschrank inkubiert und die Phagen auf Nitrocellulosefilter (Schleicher & Schüll, BA 85, Ref.-Nr. 401124) übertragen (Duplikate). Nitrocellulosefilter und Petrischalen

7

wurden mit einer Injektionskanüle markiert, um eine spätere Zuordnung zu ermöglichen. Die Petrischalen wurden während des Prozessierens der Nitrocellulosefilter im Kühlraum gelagert. Die auf den Nitrocellulosefiltern befindliche DNA wurde denaturiert, indem die Filter für 5 min. auf mit 1,5 M NaCl, 0,5 M NaOH-getränktes Filterpapier (Whatman-M3) gelegt wurden. Anschließend wurden die Filter auf die gleiche Art mit 1,5 M NaCl, 0,5 M Tris (pH 8,0) renaturiert und mit 2 x SSPE (0,36 M NaCl, 16 mM NaOH, 20 mM $NaH_2PO_4$, 2 mM EDTA) gewaschen. Die Filter wurden dann für 2 h bei 80°C im Vakuum getrocknet. Die Filter wurden für 4 h bei 65°C vorhybridisiert (Prähybridisierungslösung: 0,6 M NaCl, 0,06 M Tris (pH 8,3), 6 mM EDTA, 0,2% nichtionisches synthetisches Saccharose-Polymer ([R]Ficoll), 0,2% Polyvinylpyrrolidon 40, 0,2% BSA, 0,1% SDS, 50 μg/ml denaturierte Heringssperma-DNA). Schließlich wurden die Filter über Nacht unter Zusatz von 100 000- 200 000 Bq des markierten Oligonukleotids/ml Hybridisierungslösung (wie Prähybridisierungslösung, jedoch ohne Heringssperma-DNA) in Bechergläsern bzw. in zugeschweißten Polyethylenfolien unter leichtem Schütteln inkubiert. Die Hybridisierungstemperatur betrug 65°C. Die Nitrocellulosefilter wurden mit 6 x SSC, 0,05 M Natriumpyrophosphat eine Stunde bei Raumtemperatur und für eine weitere Stunde bei der jeweiligen Hybridisierungstemperatur ge waschen. Die Filter wurden getrocknet und über Nacht autoradiographiert. Signale auf dem Röntgenfilm, die auf beiden Duplikaten auftraten, wurden der Petrischale zugeordnet und die Region (ca. 50 Plaques) mit dem breiten Ende einer Pasteurpipette ausgestanzt und die Phagen in 1 ml SM-Puffer resuspendiert. Positive Phagen wurden über drei Runden vereinzelt, bis ein einzelner Klon vorlag.

Insgesamt wurden 1 x 10⁶ PFU der Plazenta-cDNA-Bank untersucht. Es wurden 2 weitere Signale auf Duplikat-Filtern identifiziert (Klone PP11-166 und PP11-169). Mit Hilfe des oben (S. 3) genannten 46mers aus PP11-169 wurde schließlich der Klon PP11-318 gefunden, der das gesamte 5' Ende der PP11-cDNA abdeckt. Diese 4 Klone tragen zusammen eine PP11 kodierende Sequenz wie in Tab. 2 und der Figur dargestellt.

### 10. DNA-Sequenzanalyse

Die Phagenklone (Klone PP11-93, PP11-166, PP11-169 und PP11-318) wurden vermehrt und ihre DNA jeweils extrahiert. Das jeweilige EcoRI-Fragment wurde isoliert und in die EcoRI-Stelle des Bluescript M13 Vektors (Stratagene, San Diego, CA, USA) für Restriktionsanalysen und Sequenzanalysen mittels der enzymatischen Dideoxymethode nach Sanger einliziert. Die Sequenz zeigt einen offenen Leserahmen und kodiert für ein Protein mit maximal 369 Aminosäuren. Am 5' Ende sind also 154 bp nicht translatiert, dann folgen 1107 bp kodierende Sequenz und schließlich 1138 bp untranslatierte Sequenz des 3' Endes.

### 11. Expression von PP11 in Bakterienzellen

#### a) Expression eines PP11 Fusionsproteins

pTrc99C (E. Amann et al., Gene 69 (1988) 301-315) wurde mit EcoRI und XbaI vollständig verdaut und das 4149 Basenpaar große Fragment gelelektrophoretisch isoliert. Der PP11 Klon lambda gt11-169 wurde mit EcoRI verdaut und das 1785 Basenpaar große EcoRI-Insert einschließlich Linkeranteil ebenfalls isoliert. Die PP11 cDNA des Klons lambda gt11-169 beginnt in der gezeigten Abbildung an Position 140. Das erhaltene Fragment wurde sodann mit XbaI geschnitten und das 1339 Basenpaar große EcoRI-XbaI Fragment isoliert und mit dem oben beschriebenen pTrc99C Vektorfragment ligiert. Das so erhaltene Plasmid pTrc99C-PP11 von 5488 bp ist in der Lage, in Escherichia coli Zellen die Synthese eines ca. 42 kD Proteins zu induzieren. Dieses Protein kann spezifisch mit Hilfe eines monospezifischen Kaninchen Anti-PP11 Antiserums, welches durch Immunisierung mit aus humanen Plazenten isoliertem PP11 erzeugt worden war, immungefällt werden. Ein weiterer Nachweis der PP11-Expression in Escherichia coli Zellen erfolgte mittels Western-Blot-Analysen unter Verwendung obigen Serums. In diesem Experiment reagierte nur der Extrakt aus pTrc99C-PP11 plasmidhaltigen, IPTG-induzierten Zellen, wobei wieder eine Proteinbande von ca. 42 kD spezifisch sichtbar wurde. Plasmidlose Escherichia coli Kontrollextrakte, bzw. nicht mittels IPTG induzierte pTrc99C-PP11 haltige Extrakte reagierten nicht mit dem obengenannten Anti-PP11 Antiserum. Das durch die weiter oben erzeugte Plasmidkonstruktion beschriebene PP11-Fusionsprotein hat folgende N-terminale Aminosäure sequenz, definiert durch nachfolgende Nukleotidsequenz:

```
Vektor/Linker /  5' UT Region           /    PP11

              1   2   3   4   5   6   7   8   9   1   2   3
             Met Gly Asn Ser Leu Gln Leu Gly Thr Met Arg Ala
      ...CC ATG GGG AAT TCT CTC CAA CTG GGC ACC ATG AGC GCC...
```

Das durch diese Konstruktion definierte PP11 Fusionsprotein trägt zusätzlich zu der durch die PP11 cDNA kodierte vollständige PP11 Aminosäuresequenz N-terminal vorgeschaltet neun Aminosäuren: vier vektorcodierte Aminosäuren sowie fünf Aminosäuren, die durch die in der PP11 cDNA vorkommende 5' untranslatierte Region spezifiziert werden. Zur Kontrolle wurde die weiter oben angegebene Konstruktion ebenfalls mit den Expressionsvektoren pTrc99A und pTrc99B (Deutsche Patentanmeldung P 38 19 463.5 und Amann et al. (1988) Gene 69, 301-315) durchgeführt. Diese Vektoren unterscheiden sich von pTrc99C lediglich in 2 Basenpaaren (pTrc99A) bzw. 1 Basenpaar (pTrc99B), wodurch Verschiebungen des Translations-Leserahmens hervorgerufen werden. Wie zu erwarten, waren weder pTrc99A-PP11 noch pTrc99B-PP11 in der Lage, die Synthese von mit Anti-PP11 Antiseren reagierenden PP11 Proteinen zu induzieren.

Überraschenderweise wurde gefunden, daß das durch Plasmid pTrc99C-PP11 induzierte PP11 Protein in Escherichia coli prozessiert wird. Die dem reifen PP11 Protein natürlicherweise vorgeschaltete Signalsequenz (auch "Leadersequenz" genannt) wird von E.coli Zellen erkannt und durch die Signalpeptidase gespalten. Dies kann aufgrund des Befundes geschlossen werden, daß sich nach Fraktionierung von Zellextrakten in der periplasmatischen Fraktion im Vergleich zur membranassoziierten Fraktion ein kleineres PP11 Protein nachweisen läßt, wobei der beobachtete Molekulargewichtsunterschied dem erwarteten Unterschied nach Abspalten der Signalsequenz entspricht.

b) Expression des reifen, unfusionierten Proteins:

Zur Expression des reifen, um die natürlich vorkommende Signalsequenz verkürzten PP11 Proteins wurde das weiter oben beschriebene 1339 Basenpaar lange EcoRI-XbaI-Fragment in den mit den gleichen Restriktionsenzymen verdauten Mutagenesevektor pMa5-8 ligiert. Der Mutagenesevektor pMa5-8 trägt neben einem bakteriellen Replikationsorigin und einem Antibiotikaresistenzmarker eine Klonierungs-Polylinkerregion und den Replikationsorigin des einzelsträngigen Bakteriophagen F1. Das erhaltene Plasmid pMa5-8-PP11 wurde dazu benutzt, einen Einzelstrang herzustellen. Der so erhaltene Einzelstrang der klonierten PP11 cDNA wurde dann nach bekannten Methoden isoliert und dem publizierten "gapped-duplex" Mutageneseprotokoll (Kramer et al. (1984) Nucl. Acids. Res. 12, 9441-9456) unterzogen, wobei folgendes Oligodesoxynukleotid verwendet wurde:

5' TTTGTGGTCCTCCATGGAGGCCAGGCCACA 3'

Ein Klon, der die gewünschte NcoI-Mutation aufwies (Schaffung einer neuen, in der ursprünglich isolierten cDNA nicht vorhandenen NcoI Stelle unmittelbar vor der kodierenden Sequenz für das reife PP11 Protein) wurde durch entsprechende Restriktionsanalyse identifiziert und als pMa5-8-PP11-NcoI bezeichnet. Aus diesem Plasmid wurde nach partiellem NcoI- und vollständigem XbaI-Verdau das 1268 bp große NcoI-XbaI-Fragment isoliert und in den ebenso geschnittenen Vektor pTrc99A ligiert. Das resultierende Plasmid enthält 5412 bp und exprimiert nach Induktion des trc Promotors mit IPTG das reife, unfusionierte PP11 Protein mit einem Molekulargewicht von ca. 42 kD, welches wiederum - wie bereits weiter oben beschrieben - mit Anti-PP11 Antiseren spezifisch reagiert.

12. Amidolytische Aktivität von PP11

Für PP11 konnte im folgenden colorimetrischen Testansatz eine Proteaseaktivität nachgewiesen werden. Periplasmatische oder cytoplasmatische Fraktionen von E.coli Stamm RB791 (R. Brent und M. Ptashne (1981) Proc.Natl.Acad.Sci USA 78, 4204-4208) transformiert mit pTrc 99C bzw. pTrc99C - PP11 wurden in einem optischen Test unter Verwendung des chromogenen Substrats Chromozym TH[R] (Boehringer Mannheim GmbH) gemessen. 400 $\mu$l Triäthanolamin-Puffer und 100 $\mu$l Substrat wurden mit je 300 $\mu$l des

jweiligen E.coli-Extrakts gemischt, wobei diese Extrakte auf eine Proteinkonzentration von 16 mg/ml verdünnt waren. Die Reaktionsküvette wurde bei 37°C inkubiert und die Absorptions-Änderung pro Minute bei 405 nm aufgezeichnet. In einigen Experimenten wurde der E.coli Extrakt und Kontroll-Extrakt mit einem Anti-PP11-Antiserum von Kaninchen vorinkubiert und bei der Berechnung der amidolytischen Aktivitäten der Extrakte die amidolytische Aktivität des Antiserums subtrahiert. Bei gleicher Konzentration von Gesamtprotein bewirkte der PP11 enthaltende E.coli Extrakt eine höhere Absorptionszunahme ( A = 0.04 pro Minute) als die Kontrolle ( A = 0.024 pro Minute) entsprechend 32 mU/ml bzw. 19.2 mU/ml. Die amidolytische Aktivität von PP11 wurde fast völlig durch Vorinkubation mit Anti-PP11 Antiserum gehemmt. Ferner zeigte das cytoplasmatische unprozessierte PP11 mit dem Molekulargewicht von 45 kD diese amidolytische Aktivität nicht. Da die amidolytische Aktivität durch Diisopropylfluorophosphat gehemmt wird, handelt es sich bei PP11 wahrscheinlich um eine Serinprotease.

```
        10                    30                    50
CTTCCTGAAAGGATCTGGAGACACCAGCTCCACAAGTCCTGGTGTCTTTAAAAGGATCAG

        70                    90                    110
CTTGAGGAATAAGGCTCGTCTGAGAGCTGTGACATTCATCTGACTCTAGTGAAAGTCCAA

        130                   150                   170
CAGCCACTCCCTTTTTGGCCTCCAACTGGGCACCATGAGGGCCTGCATCTCCCTGGTATT
                                      M   R   A   C   I   S   L   V   L

        190                   210                   230
GGCCGTGCTGTGTGGCCTGGCCTGGGCTGAGGACCACAAAGAGTCAGAGCCATTGCCACA
A   V   L   C   G   L   A   W   A   E   D   H   K   E   S   E   P   L   P   Q

        250                   270                   290
GCTGGAGGAAGAGACAGAAGAGGCCCTCGCCAGCAACTTGTACTCGGCACCCACCTCCTG
  L   E   E   E   T   E   E   A   L   A   S   N   L   Y   S   A   P   T   S   C

        310                   330                   350
CCAGGGCCGCTGCTACGAAGCCTTTGACAAGCACCACCAATGTCACTGCAATGCCCGCTG
  Q   G   R   C   Y   E   A   F   D   K   H   H   Q   C   H   C   N   A   R   C

        370                   390                   410
CCAAGAGTTTGGGAACTGCTGCAAGGATTTTGAGAGCCTGTGTAGTGACCACGAGGTCTC
  Q   E   F   G   N   C   C   K   D   F   E   S   L   C   S   D   H   E   V   S

        430                   450                   470
CCACAGCAGTGATGCCATAACAAAAGAGGAGATTCAGAGCATCTCTGAGAAGATCTACAG
  H   S   S   D   A   I   T   K   E   E   I   Q   S   I   S   E   K   I   Y   R

        490                   510                   530
GGCAGACACCAACAAAGCCCAGAAGGAAGACATCGTTCTCAATAGCCAAAACTGCATCTC
  A   D   T   N   K   A   Q   K   E   D   I   V   L   N   S   Q   N   C   I   S

        550                   570                   590
CCCGTCAGAGACCAGAAACCAAGTGGATCGCTGCCCAAAGCCACTCTTCACTTATGTCAA
  P   S   E   T   R   N   Q   V   D   R   C   P   K   P   L   F   T   Y   V   N

        610                   630                   650
TGAGAAGCTGTTCTCCAAGCCCACCTATGCAGCCTTCATCAACCTCCTCAACAACTACCA
  E   K   L   F   S   K   P   T   Y   A   A   F   I   N   L   L   N   N   Y   Q

        670                   690                   710
GCGGGCAACAGGCCATGGGGAGCACTTCAGTGCCCAGGAGCTGGCCGAGCAGGACGCCTT
  R   A   T   G   H   G   E   H   F   S   A   Q   E   L   A   E   Q   D   A   F

        730                   750                   770
CCTCAGAGAGATCATGAAGACAGCAGTCATGAAGGAGCTCTACAGCTTCCTCCATCACCA
  L   R   E   I   M   K   T   A   V   M   K   E   L   Y   S   F   L   H   H   Q

        790                   810                   830
GAATCGCTATGGCTCAGAGCAAGAGTTTGTCGATGACTTGAAGAACATGTGGTTTGGGCT
  N   R   Y   G   S   E   Q   E   F   V   D   D   L   K   N   M   W   F   G   L
```

Tab. 2

```
        850                870                890
CTATTCGAGAGGCAATGAAGAGGGGGACTCGAGTGGCTTTGAACATGTCTTCTCAGGTGA
  Y   S   R   G   N   E   E   G   D   S   S   G   F   E   H   V   F   S   G   E

        910                930                950
GGTAAAAAAAGGCAAGGTTACTGGCTTCCATAACTGGATCCGCTTCTACCTGGAGGAGAA
  V   K   K   G   K   V   T   G   F   H   N   W   I   R   F   Y   L   E   E   K

        970                990                1010
GGAGGGTCTGGTTGACTATTACAGTCACATCTACGATGGGCCTTGGGATTCTTACCCCGA
  E   G   L   V   D   Y   Y   S   H   I   Y   D   G   P   W   D   S   Y   P   D

        1030               1050               1070
TGTGCTGGCAATGCAGTTCAACTGGGACGGCTACTATAAGGAAGTGGGCTCTGCTTTCAT
  V   L   A   M   Q   F   N   W   D   G   Y   Y   K   E   V   G   S   A   F   I

        1090               1110               1130
CGGCAGCAGCCCTGAGTTTGAGTTTGCACTCTACTCCCTGTGCTTCATCGCCAGGCCAGG
  G   S   S   P   E   F   E   F   A   L   Y   S   L   C   F   I   A   R   P   G

        1150               1170               1190
CAAAGTGTGCCAGTTAAGCCTGGGAGGATATCCCTTAGCTGTCCGGACATATACCTGGGA
  K   V   C   Q   L   S   L   G   G   Y   P   L   A   V   R   T   Y   T   W   D

        1210               1230               1250
CAAGTCCACCTATGGGAATGGCAAGAAGTACATCGCCACAGCCTACATAGTGTCTTCCAC
  K   S   T   Y   G   N   G   K   K   Y   I   A   T   A   Y   I   V   S   S   T

        1270               1290               1310
CTAATAGAACTTCGAGCCAGAAAGGGGCATGAGGGCTCTTGCGAGACTGAAGTGCTATCT

        1330               1350               1370
TCTCTGGACTAGAGAGAAGAGGGAGAGGACTGGAAGGGATCACCAAATCTCAAAGCAATG

        1390               1410               1430
AGAAGCATTCCTAAATCCCAAAGTGCCCACATGGGAAAGAGATAAAATGTACAAATTAGA

        1450               1470               1490
AAAATGTGGATAAACAGTCAAACCTTTATCCTCTAGAATTTTGGCAATGTTGACTAAGAA

        1510               1530               1550
ACAGAGTCCAAGCAGAGAAGGTAGGAACCCTCCATAGCTCTCTGCCCTGATGTGTGGGGG

        1570               1590               1610
AACTAGGAAGAAGTCCTTTGACCTCACCAGGCCTCATGCTTCCCTTTAATGTAAAGGGAA

        1630               1650               1670
GGGGTTTGCCCACTTTCCTCTTTTTGGGGTTGGTGAGAGGGCAAACCCTGATATTTTTAC

        1690               1710               1730
TGTGAAGGTGTTTTCAGTTGTTCTTAGGAAGAACAGCTGATAGAAATTCAAGATTACTAT

        1750               1770               1790
AATGGCTGTTATTATACACAGCTCTGTAAACTACCACTCAGCCCTGTGTTGGGGTCCTCA
```

Tab. 2 ff

```
        1810              1830              1850
AAGAAGTAAGGCCACAGTAATCAAGCAAGGGCCTTTGGTTTTTTCCAGAGTTAGATCCTC

        1870              1890              1910
TCAGAACAGAGTCTGGGAGAACTCCAATGCTGAATGGAGAAGGGTAATAGGTTGGTTGCA

        1930              1950              1970
GTGAATGGGCTGGGGGTGGGGTGGCCTTCTCCAGGCCTGAGTGTTTTTGTGTCCAGCTCA

        1990              2010              2030
GTATCTGCAACAAGAAGTTTCCCACTTGTGGATGTTTAGTGCAGCCACAGACTTGTATTT

        2050              2070              2090
TGATCCCCAATTTTTTTTGAAAGAGTTCTCCTCATAGGAGGATGATTCAGCATCAGAAGA

        2110              2130              2150
AGAAGGAACCCATAGCTTGGTGTCATTAACATAATTATTTTAAGCCTTATCCAGCAGCCA

        2170              2190              2210
TAATTTGAATAACTCTACGAGACCAGAGAGACTGTAGTTCCCTATTTTAACCTCAATTAT

        2230              2250              2270
GCATTTGTCCCCAACCCCACTGAGAACTAAATGCTGTACCACAGAGCCGGGTGTGAACTA

        2290              2310              2330
TGGTTTAGAAGGTTCAAGTTTCCAATTAAAGTCATTGAAGAAAAAAAAAAAAAAAAAAAA

        2350              2370              2390
AAAAAAAAAAAAAAAAAAAAAAAAAAAAAAAAAAAAAAAAAAAAAAAAAAAAAAAAAAAAA
```

Tab. 2 ff

**Ansprüche**

1. DNA-Sequenz, kodierend für die Aminosäuresequenz gemäß Tabelle 2, ihre Allele und Varianten.

2. DNA-Sequenz, kodierend für plazentaspezifisches Protein PP11, enthaltend den kodierenden Strang gemäß Tabelle 2.

3. DNA oder RNA, die mit der DNA gemäß Anspruch 1 unter stringenten Bedingungen hybridisiert.

4. Genstruktur, enthaltend eine DNA oder RNA gemäß Anspruch 1, 2 oder 3.

5. Vektor, enthaltend eine DNA oder RNA nach einem oder mehreren der vorhergehenden Ansprüche.

6. Transformierte Zellen, enthaltend DNA oder RNA nach Anspruch 1, 2, 3, 4 oder 5.

7. Gentechnisch erhältliches PP11, gekennzeichnet durch die Aminosäuresequenz gemäß Tabelle 2.

8. PP11, dadurch gekennzeichnet, daß es in E.coli durch Expression der DNA-Sequenz nach Anspruch 2 gentechnisch hergestellt ist.

9. PP11, dadurch gekennzeichnet, daß es in Hefe durch Expression der DNA-Sequenz nach Anspruch 2 gentechnisch hergestellt ist.

10. Verfahren zur Herstellung von PP11, dadurch gekennzeichnet, daß man eine cDNA oder RNA nach Anspruch 1, 2, 3 oder 4, in ein Expressionssystem einbringt und dort zur Expression bringt.

11. Für PP11 spezifische polyklonale oder monoklonale Antikörper, erhalten aus gentechnisch hergestelltem PP11 bzw. antigenwirksamen Teilen davon.

12. Diagnostikum, das eine DNA oder RNA nach Anspruch 1, 2, 3 oder 4 ganz oder teilweise enthält.

13. Diagnostikum, das eine DNA oder RNA ganz oder teilweise enthält, die zu der DNA nach Anspruch 10 komplementär ist.

14. Diagnostikum, enthaltend Antikörper nach Anspruch 9.

15. Diagnostizierverfahren, dadurch gekennzeichnet, daß Körperflüssigkeiten, Gewebe oder daraus isolierte Nukleinsäuren mit einem Diagnostikum nach Anspruch 10, 11 oder 12 in Kontakt gebracht werden.

16. PP11 zur Verwendung als Arzneimittel.

17. Arzneimittel, gekennzeichnet durch einen Gehalt an nach Anspruch 7 hergestelltem PP11.

Patentansprüche für den folgende Vertragsstaaten: ES, GR

1. Verfahren zur Herstellung von PP11, dadurch gekennzeichnet, daß man eine DNA-Sequenz kodierend für die Aminosäuresequenz gemäß Tabelle 2, in ein Expressionssystem einbringt und dort zur Expression bringt.

2. Verfahren nach Anspruch 1, dadurch gekennzeichnet, daß eukaryotische Zellen als Expressionssystem eingesetzt werden.

3. Verfahren nach Anspruch 2, dadurch gekennzeichnet, daß Hefe eingesetzt wird.

4. Verfahren zur Herstellung eines Diagnostikums, dadurch gekennzeichnet, daß die DNA Sequenz kodierend für PP11, seine Allele, Varianten und Mutanten eingesetzt werden.

5. Verfahren zur Herstellung eines Arzneimittels, dadurch gekennzeichnet, daß die DNA-Sequenz von PP11 gemäß Tabelle 2 in einem Expressionssystem zur Expression gebracht wird, das PP11 gereinigt und mit üblichen Trägermaterialien gemischt wird.